# EUROPEAN PATENT SPECIFICATION

(11) **EP 2 821 002 B1**
(45) Date of publication and mention of the grant of the patent: **22.06.2016**
(21) Application number: 13874437.0
(22) Date of filing: 05.12.2013
(51) Int. Cl.: A61B 1/00, G02B 23/24, A61B 1/12

(54) **STEREOSCOPIC ENDOSCOPE**
STEREOSKOPISCHES ENDOSKOP
ENDOSCOPE STÉRÉOSCOPIQUE

(30) Priority: 06.02.2013 JP 2013021577
(43) Date of publication of application: 07.01.2015
(73) Proprietor: Olympus Corporation, Shibuya-ku Tokyo (JP)
(72) Inventor: AONO Susumu, Tokyo 151-0072 (JP)
(74) Representative: Schicker, Silvia
(86) International application number: PCT/JP2013/082724
(87) International publication number: WO 2014/122843

(56) References cited:
- JP-A- H06 154 155
- JP-A- 2000 262 463
- JP-A- 2000 262 463
- JP-A- 2008 148 935
- JP-A- 2009 028 198
- JP-A- 2012 045 327
- JP-U- H01 133 901
- JP-U- H01 133 901
- US-A- 6 142 932

## Description

### Technical Field

The present invention relates to a stereoscopic endoscope that picks up a stereoscopic image of a region to be examined in a subject.

### Background Art

In recent years, a so-called stereoscopic endoscope that picks up a stereoscopic image of a region to be examined in a subject is well known. In the stereoscopic endoscope, first and second objective lenses are respectively provided on a distal end face of an insertion portion. In the stereoscopic endoscope, first and second image pickup devices, on which the image of the region to be examined is formed respectively via the respective objective lenses, are provided in a distal end in an inserting direction (hereinafter simply referred to as distal end) of the insertion portion.

An external apparatus, to which the stereoscopic endoscope is connected, repeatedly displays, on a monitor, a first observation image formed on the first image pickup device and a second observation image formed on the second image pickup device. An observer observers, via dedicated stereoscopic vision glasses, the first observation image and the second observation image repeatedly displayed on the monitor in such a manner as to observe only the first observation image with one eye and observe only the second observation image with the other eye. Consequently, the observer can stereoscopically view the region to be examined.

When the insertion portion is inserted into the subject, it is likely that stains adhere to lens surfaces of the respective objective lenses provided on the distal end face or fog occurs. If stains adhere to the lens surfaces of the respective objective lenses or fog occurs, it is likely that the region to be examined is seen differently in the respective observation images.

Therefore, Japanese Patent Application Laid-Open Publication No. H6-154155 discloses a configuration in which one or a pair of nozzle portions is provided on a distal end face of an insertion portion. The one or the pair of nozzle portions described in Japanese Patent Application Laid-Open Publication No. H6-154155 supplies fluid to lens surfaces of respective objective lenses to thereby remove stains or fog of the lens surfaces of the respective objective lenses.

Japanese Patent Application Laid-Open Publication No. 2000-10022 also discloses a configuration in which a pair of nozzle portions is provided on a distal end face of an insertion portion. The pair of nozzle portions described in Japanese Patent Application Laid-Open Publication No. 2000-10022 supplies fluid respectively to lens surfaces of respective objective lenses to thereby remove stains or fog of the lens surfaces of the respective objective lenses.

However, in the configuration in which the pair of nozzle portions is provided on the distal end face disclosed in Japanese Patent Application Laid-Open Publication No. H6-154155, a direction in which the fluid is supplied to the lens surface of a first objective lens from a first nozzle portion and a direction in which the fluid is supplied to a second objective lens from a second nozzle portion are different.

Therefore, during observation of a region to be examined, when the fluid is supplied to the lens surfaces of the respective objective lenses from the respective nozzle portions, directions of the fluid flowing on the lens surfaces of the respective objective lenses are different. Accordingly, there is a problem in that the region to be examined is seen differently in respective observation images and an unnatural stereoscopic image is formed and, as a result, eyes of an observer get tired.

In the configuration in which the one nozzle portion is provided on the distal end face disclosed in Japanese Patent Application Laid-Open Publication No. H6-154155, supply directions of the fluid to the respective objective lenses are the same.

However, in the first objective lens located near the nozzle portion and the second objective lens located apart from the nozzle portion, supply speeds and supply strengths of the fluid supplied from the nozzle portions are different. That is, the fluid is not uniformly supplied to the lens surfaces of the respective objective lenses. Therefore, likewise, there is a problem in that the region to be examined is seen differently in the respective observation images and an unnatural stereoscopic image is formed and, as a result, the eyes of the observer get tired.

Further, the fluid is supplied to the lens surfaces of the respective objective lenses from sides equivalent to a left-right direction of the respective observation images. As a result, the observer views an image in which the fluid flows in the left-right direction of the respective observation images. In general, it feels natural when the fluid flows in an up-down direction. Therefore, there is also a problem in that the observer feels a sense of discomfort concerning the image in which the fluid flows in the left-right direction.

In the configuration in which the pair of nozzle portions is provided disclosed in Japanese Patent Application Laid-Open Publication No. 2000-10022, similarly, a direction in which the fluid is supplied to the first objective lens from the first nozzle portion and a direction in which the fluid is supplied to the second objective lens from the second nozzle portion are different. Specifically, the directions are different by 180°.

Therefore, during the observation of the region to be examined, when the fluid is supplied to the lens surfaces of the respective objective lenses from the respective nozzle portions, the directions of the fluid flowing on the lens surfaces of the respective objective lenses are different by 180°.

Therefore, there is a problem in that the region to be examined is seen differently in the respective observation images and an unnatural stereoscopic image is obtained and, as a result, eyes of the observer get tired as in Japanese Patent Application Laid-Open Publication No. H6-154155.

The present invention has been devised in view of the problems and it is an object of the present invention to provide a stereoscopic endoscope with which the observer can easily perform the observation even when stains and fog of the lens surfaces of the respective objective lenses are removed by the supply of the fluid during the observation of the respective observation images.

### Disclosure of Invention

### Means for Solving the Problem

A stereoscopic endoscope according to an aspect of the present invention includes: an insertion portion inserted into a subject; a first observation window provided at a distal end portion on an inserting direction distal end side of the insertion portion; a second observation window provided at the distal end portion; a first delivery portion provided a predetermined distance apart from an optical axis center of the first observation window and configured to deliver fluid to the first observation window; and a second delivery portion provided a distance equivalent to the predetermined distance apart from an optical axis center of the second observation window and configured to deliver the fluid in a supply direction same as a supply direction of the first delivery portion to the second observation window.

### Brief Description of the Drawings

Fig. 1 is a diagram schematically showing a configuration of an endoscope apparatus including a stereoscopic endoscope of a first embodiment;
Fig. 2 is a block diagram of the endoscope apparatus shown in Fig. 1;
Fig. 3 is a diagram showing, side by side, a state in which a first observation image or a second observation image of a region to be examined is displayed on a display unit shown in Fig. 1 and Fig. 2 and a state in which the image of the region to be examined is formed as the first observation image or the second observation image on a first image pickup device or a second image pickup device;
Fig. 4 is an enlarged plan view of a distal end face of an insertion portion surrounded by an IV line in Fig. 1;
Fig. 5 is a diagram showing a distal end side of the insertion portion of the endoscope along a V-V line in Fig. 4 partially as a cross section;
Fig. 6 is a diagram showing the distal end side of the insertion portion of the endoscope along a VI-VI line in Fig. 4 partially as a cross section;
Fig. 7 is a diagram showing a modification of a conduit configuration for supplying fluid to a first nozzle portion and a second nozzle portion shown in Fig. 6 together with the distal end side of the insertion portion of the endoscope partially as a cross section;
Fig. 8 is a partial sectional view showing an overview of a configuration of an air feeding and water feeding switching device provided in an operation portion of the endoscope in Fig. 1;
Fig. 9 is an enlarged plan view of the distal end face of the insertion portion showing a modification in which, on the distal end face shown in Fig. 4, the first nozzle portion and the second nozzle portion are located by being shifted in a direction crossing, at a set angle, a direction connecting a side in an up direction and a side in a down direction of the first image pickup device and the second image pickup device;
Fig. 10 is an enlarged plan view of the distal end face of the insertion portion showing a modification in which arrangement positions of a first illumination lens and a second illumination lens on the distal end face shown in Fig. 4 are determined with reference to a first objective lens and a second objective lens;
Fig. 11 is a plan view showing a distal end face of an insertion portion of a stereoscopic endoscope of a second embodiment together with a distal end face of a sheath;
Fig. 12 is a partial sectional view showing a distal end side of the insertion portion along a XII-XII line in Fig. 11 together with the sheath;
Fig. 13 is a plan view showing a configuration of a modification of nozzle portions shown in Fig. 11 together with the distal end face of the insertion portion of the stereoscopic endoscope and the distal end face of the sheath;
Fig. 14 is a partial sectional view showing the distal end side of the insertion portion along a XIV-XIV line in Fig. 13 together with the sheath; and
Fig. 15 is an enlarged plan view of the distal end face of the insertion portion showing a modification in which a first opening portion and a second opening portion shown in Fig. 4 are provided in one nozzle portion located on the distal end face of the insertion portion.

### Best Mode for Carrying Out the Invention

Embodiments of the present invention are explained below with reference to the drawings.

### (First Embodiment)

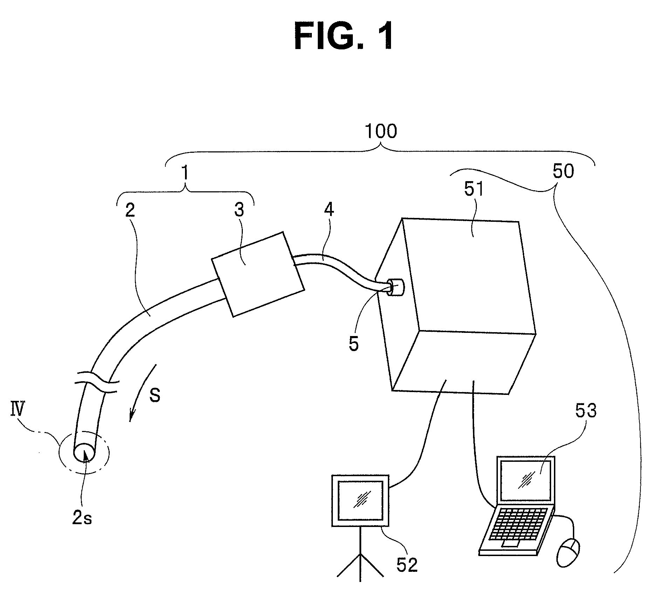

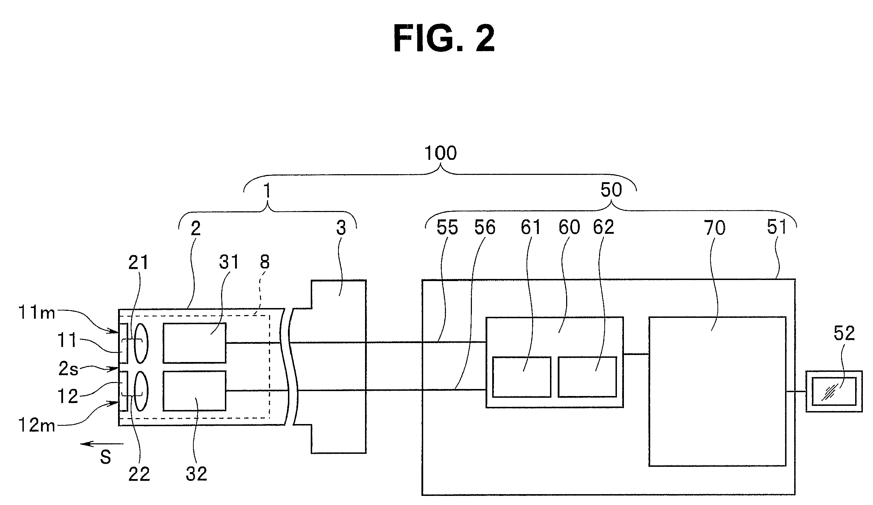

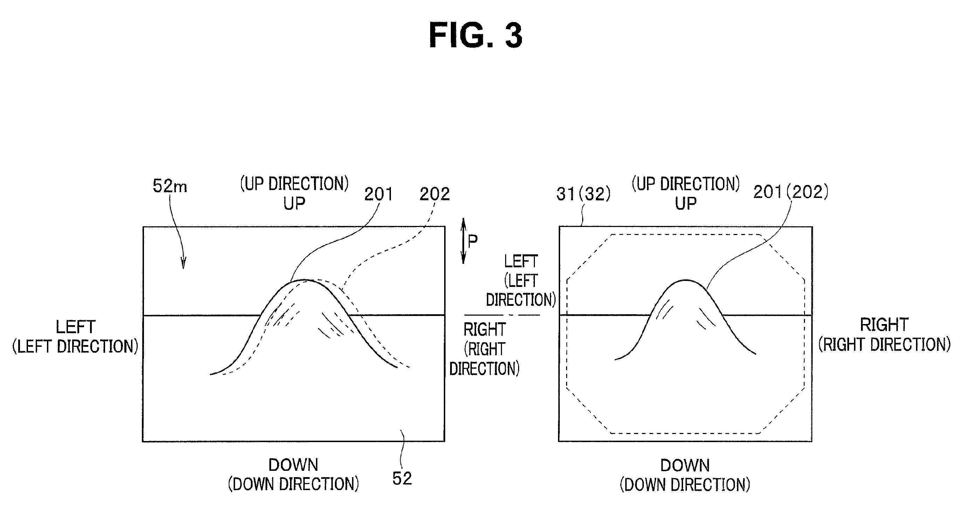

Fig. 1 is a diagram schematically showing a configuration of an endoscope apparatus including a stereoscopic endoscope of an embodiment. Fig. 2 is a block diagram of the endoscope apparatus shown in Fig. 1. Fig. 3 is a diagram showing, side by side, a state in which a first observation image or a second observation image of a region to be examined is displayed on a display unit shown in Fig. 1 and Fig. 2 and a state in which the image of the region to be examined is formed as the first observation image or the second observation image on a first image pickup device or a second image pickup device.

As shown in Fig. 1 and Fig. 2, in an endoscope apparatus 100, a main part is configured to include a stereoscopic endoscope 1 that picks up a stereoscopic image of a region to be examined in a subject and peripheral apparatuses 50.

The stereoscopic endoscope 1 includes an insertion portion 2 inserted into the subject and an operation portion 3 connected to a proximal end in an inserting direction S (hereinafter simply referred to as proximal end) of the insertion portion 2. The stereoscopic endoscope 1 includes a universal cord 4 extended from the operation portion 3 and a connector 5 provided at an extension end of the universal cord 4.

Note that, in Fig. 1, the stereoscopic endoscope 1 is exemplified by a flexible endoscope in which the insertion portion 2 has flexibility. However, the stereoscopic endoscope 1 may be a rigid endoscope in which the insertion portion 2 is rigid.

The peripheral apparatuses 50 include an image processing unit 51, a display unit 52 such as a 3D monitor connected to the image processing unit 51, and an input unit 53 connected to the image processing unit 51.

Note that the stereoscopic endoscope 1 is connectable to the image processing unit 51 because the connector 5 is detachably attachable to the image processing unit 51.

As shown in Fig. 2, an image pickup unit 8 that picks up an image of the region to be examined is provided in a distal end in the inserting direction S (hereinafter simply referred to as distal end) of the insertion portion 2. The image pickup unit 8 includes a first objective optical system 21 that observes the region to be examined and a second objective optical system 22 that observes the region to be examined.

Further, the image pickup unit 8 includes, as shown in Fig. 3, a first image pickup device 31 on which the image of the region to be examined is formed as a first observation image 201 via the first objective optical system 21. The image pickup unit 8 includes a second image pickup device 32 on which the image of the region to be examined is formed as a second observation image 202, which has a parallax from the first observation image 201, via the second objective optical system 22.

That is, the image pickup unit 8 includes a function of picking up the first observation image 201 and the second observation image 202 having the parallax from the first observation image 201.

In the first objective optical system 21, a lens located front most in the inserting direction S is located as a first objective lens 11, which is a first observation window, such that a lens surface 11m is exposed on a distal end face 2s of a distal end of the insertion portion 2. Note that the first observation window is not limited to the objective lens and may be a cover glass or the like. The objective lens is explained as an example of the first observation window below.

In the second objective optical system 22, a lens located front most in the inserting direction S is located as a second objective lens 12, which is a second observation window, such that a lens surface 12m is exposed on the distal end face 2s. Note that the second observation window is not limited to the objective lens and may be a cover glass or the like. The objective lens is explained as an example of the second observation window below.

Note that arrangement positions of the first objective lens 11 and the second objective lens 12 on the distal end face 2s and other members provided on the distal end face 2s are explained below.

In the distal end of the insertion portion 2, the first image pickup device 31 is located behind the first objective optical system 21 in the inserting direction S. Further, in the distal end of the insertion portion 2, the second image pickup device 32 is located behind the second objective optical system 22 in the inserting direction S.

A distal end of a first image pickup cable 55 is connected to the first image pickup device 31. A distal end of the second image pickup cable 56 is connected to the second image pickup device 32.

The first image pickup cable 55 and the second image pickup cable 56 are inserted through the insertion portion 2 and the operation portion 3. When the connector 5 is connected to the image processing unit 51, respective proximal ends of the first image pickup cable 55 and the second image pickup cable 56 are connected to a picked-up image generating section 60 of the image processing unit 51.

The picked-up image generating section 60 includes a first image generating section 61 and a second image generating section 62. The first image generating section 61 generates the first observation image 201 formed on the first image pickup device 31. The second image generating section 62 generates the second observation image 202 formed on the second image pickup device 32.

The picked-up image generating section 60 is connected to a stereoscopic display processing section 70. Further, the stereoscopic display processing section 70 is connected to the display unit 52.

As shown in Fig. 3, the stereoscopic display processing section 70 alternately repeatedly displays the first observation image 201 and the second observation image 202 on the display unit 52. An observer observers, via dedicated stereoscopic vision glasses, the first observation image 201 and the second observation image 202 repeatedly displayed on the display unit 52 in such a manner as to observe only the first observation image 201 with a left eye and observe only the second observation image 202 with a right eye. Consequently, the observer can stereoscopically view the region to be examined.

Note that, in the following explanation, in the present embodiment, as shown in Fig. 3, up and down and left and right directions of the first observation image 201 and the second observation image 202 formed on the first image pickup device 31 and the second image pickup device 32 are defined as up and down and left and right directions.

Specifically, in a state in which a down direction in a gravity direction and a down direction of the region to be examined coincide with each other, when the first objective lens 11 and the second objective lens 12 view the region to be examined in front, up and down and left and right directions of the first observation image 201 formed on the first image pickup device 31 via the first objective lens 11 and the second observation image 202 formed on the second image pickup device 32 via the second objective lens 12 are defined as up and down and left and right directions.

In this case, the down direction in the gravity direction of the distal end face 2s is defined as a down direction (represented as DOWN in Figs. 3 to 5 and 9 to 14). An opposite direction of the down direction of the distal end face 2s is defined as an up direction (represented as UP in Figs. 3 to 5 and 9 to 14). When the distal end face 2s is viewed in a direction of the inserting direction S, a direction 90 degrees clockwise from the up direction is defined as a right direction (represented as RIGHT in Figs. 3, 4, 6, 7, 9 to 11, and 13). An opposite direction of the right direction is defined as a left direction (represented as LEFT in Figs. 3, 4, 6, 7, 9 to 11, and 13).

As shown in Fig. 3, the up and down and left and right directions defined by the first image pickup device 31 or the second image pickup device 32 coincide with up and down and left and right directions of the first observation image 201 or the second observation image 202 displayed on a display surface 52m with a position thereof adjusted by a display position adjusting section 73.

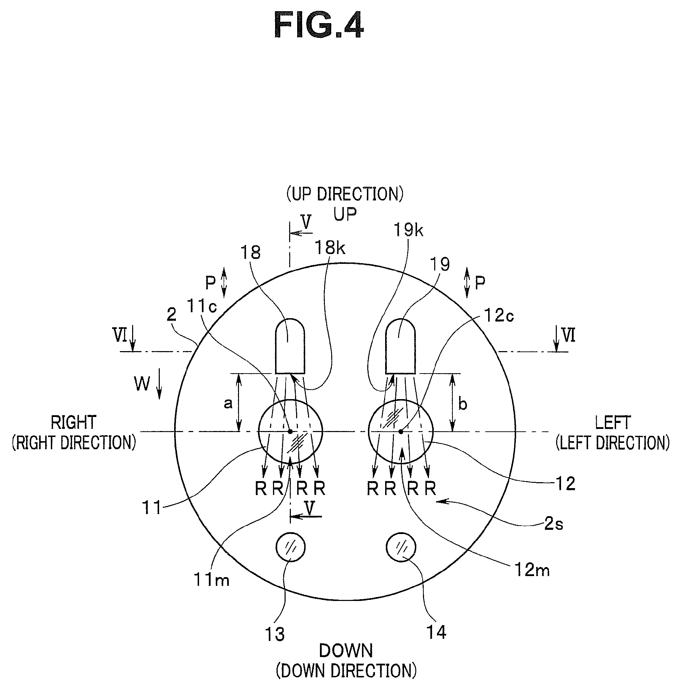

Next, a configuration of the distal end face 2s of the insertion portion 2 is explained with reference to Fig. 4. Fig. 4 is an enlarged plan view of the distal end face of the insertion portion surrounded by an IV line in Fig. 1.

As shown in Fig. 4, the first objective lens 11 and the second objective lens 12 are provided on the distal end face 2s at a distal end portion of the insertion portion 2. Note that the first objective lens 11 and the second objective lens 12 are coaxially provided a set interval apart from each other.

On the distal end face 2s, a first nozzle portion 18 is provided further in the up direction than the first objective lens 11.

The first nozzle portion 18 delivers fluid R to the lens surface 11m of the first objective lens 11 from a first opening portion 18k, which is a first delivery portion, located to be opposed to an outer circumference of the first objective lens 11 and located a predetermined distance "a" apart from an optical center 11c of the first objective lens 11.

Further, on the distal end face 2s, a second nozzle portion 19 is provided further in the up direction than the second objective lens 12.

The second nozzle portion 19 delivers the fluid R to the lens surface 12m of the second objective lens 12 from a second opening portion 19k, which is a second delivery portion, located to be opposed to an outer circumference of the second objective lens 12 and located a predetermined distance "b" apart from an optical center 12c of the second objective lens 12.

Note that an opening axis direction of the first opening portion 18k and an opening axis direction of the second opening portion 19k are located in parallel to each other. The distance "a" of the first opening portion 18k from the optical center 11c of the first objective lens 11 and the distance "b" of the second opening portion 19k from the optical center 12c of the second objective lens 12 coincide with each other (a=b) or substantially coincide with each other (a≈b).

A direction in which the fluid R is delivered to the lens surface 12m from the second opening portion 19k coincides with a supply direction W of the fluid R delivered to the lens surface 11m from the first opening portion 18k.

That is, in the present embodiment, the supply direction W of the fluid R coincides with a direction parallel to a direction P connecting a side in the up direction and a side in the down direction of the first image pickup device 31 and the second image pickup device 32.

Consequently, the fluid R is supplied to the lens surface 11m of the first objective lens 11 and the lens surface 12m of the second objective lens 12 from the up direction. As a result, the fluid R flows to the lens surfaces 11m and 12m from the up direction to the down direction.

That is, as shown in Fig. 3, the observer observing the display unit 52 with the dedicated stereoscopic vision glasses on can observe the display unit 52 in a state in which the fluid R is supplied from the up direction in both of the first observation image 201 observed by the right eye and the second observation image 202 observed by the left eye.

Note that any one of water repellent coating and hydrophilic coating may be applied to the lens surfaces 11m and 12m. Consequently, the fluid is more easily removed from the respective lens surfaces 11m and 12m. Therefore, observability is improved.

The first nozzle portion 18 and the second nozzle portion 19, that is, the first opening portion 18k and the second opening portion 19k may be respectively provided apart from each other by the distances "a" and "b" respectively from the first objective lens 11 and the second objective lens 12 in the down direction on the distal end face 2s as long as the supply direction W of each fluid R coincides with the direction P.

Accordingly, on the distal end face 2s, that first nozzle portion 18 and the second nozzle portion 19, that is, the first opening portion 18k and the second opening portion 19k only have to be provided in the up direction or the down direction apart from the first objective lens 11 and the second objective lens 12 respectively by the distances "a" and "b".

As explained above, when the first nozzle portion 18 and the second nozzle portion 19, that is, the first opening portion 18k and the second opening portion 19k are provided in any one of the left and right directions apart from the first objective lens 11 and the second objective lens 12 respectively by distances "a" and "b", the fluid R is supplied to the lens surfaces 11m and 12m of the respective objective lenses 11 and 12 from the left and right direction sides. As a result, it is likely that the observer observing the first observation image 201 or the second observation image 202 displayed on the display unit 52 feels a sense of discomfort. However, if the respective nozzle portions 18 and 19, that is, the first opening portion 18k and the second opening portion 19k are provided in any one of the up and down directions with respect to the respective objective lenses 11 and 12, the observer does not feel a sense of discomfort involved in the supply of the fluid R.

Usually, when the fluid R is supplied downward in the gravity direction, the fluid R is easily supplied from the respective nozzle portions 18 and 19. Accordingly, on the distal end face 2s, it is preferable that the first nozzle portion 18 and the second nozzle portion 19, that is, the first opening portion 18k and the second opening portion 19k are provided in the up direction with respect to the first objective lens 11 and the second objective lens 12 rather than in the down direction.

Note that a flow rate of the fluid R per unit time supplied to the lens surface 11m of the first objective lens 11 from the first opening portion 18k and a flow rate of the fluid R per unit time supplied to the lens surface 12m of the second objective lens 12 from the second opening portion 19k are set to be equal or substantially equal to each other.

Further, on the distal end face 2s, as shown in Fig. 4, when the first nozzle portion 18 and the second nozzle portion 19 are provided in the up direction with respect to the first objective lens 11 and the second objective lens 12, a first illumination lens 13 and a second illumination lens 14, which illuminate an inside of the subject, are respectively provided a set interval apart in the down direction from the first objective lens 11 and the second objective lens 12.

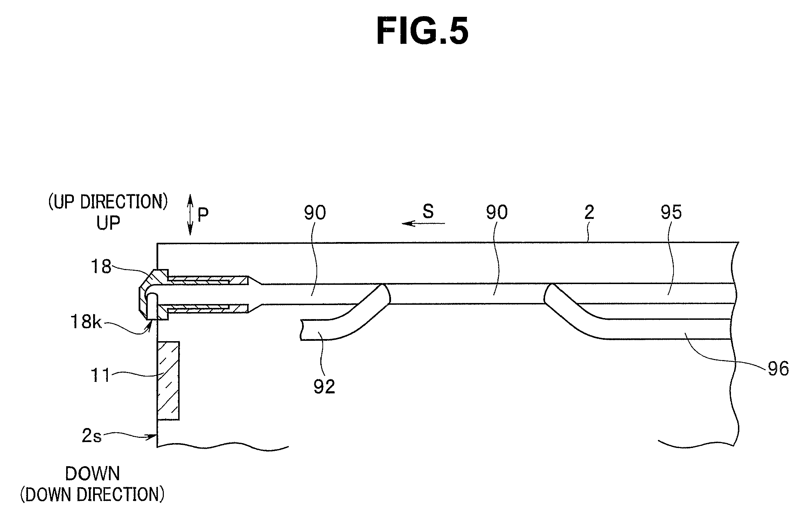

Next, a configuration for supplying the fluid R to the first nozzle portion 18 and the second nozzle portion 19 is briefly explained with reference to Fig. 5 to Fig. 8.

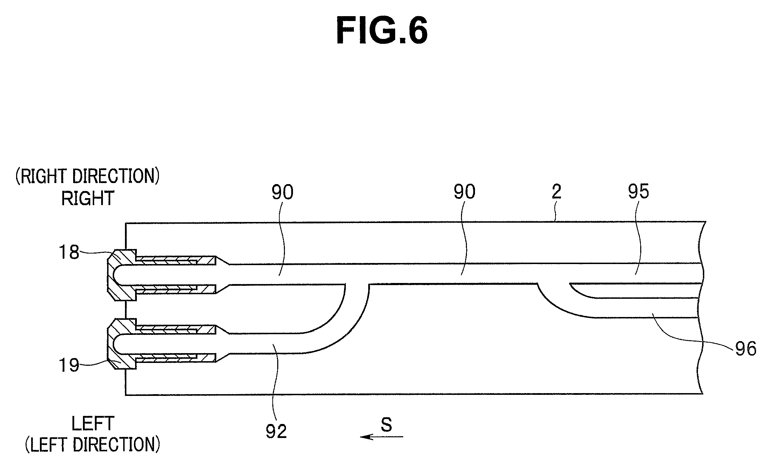

Fig. 5 is a diagram showing a distal end side of the insertion portion of the endoscope along a V-V line in Fig. 4 partially as a cross section. Fig. 6 is a diagram showing the distal end side of the insertion portion of the endoscope along a VI-VI line in Fig. 4 partially as a cross section.

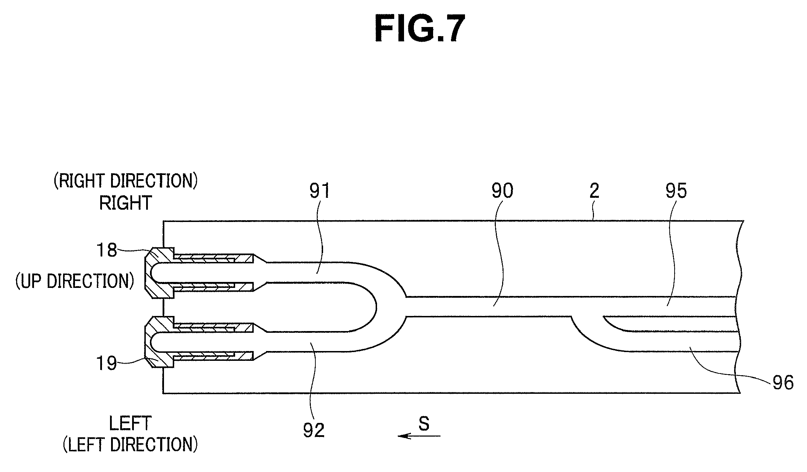

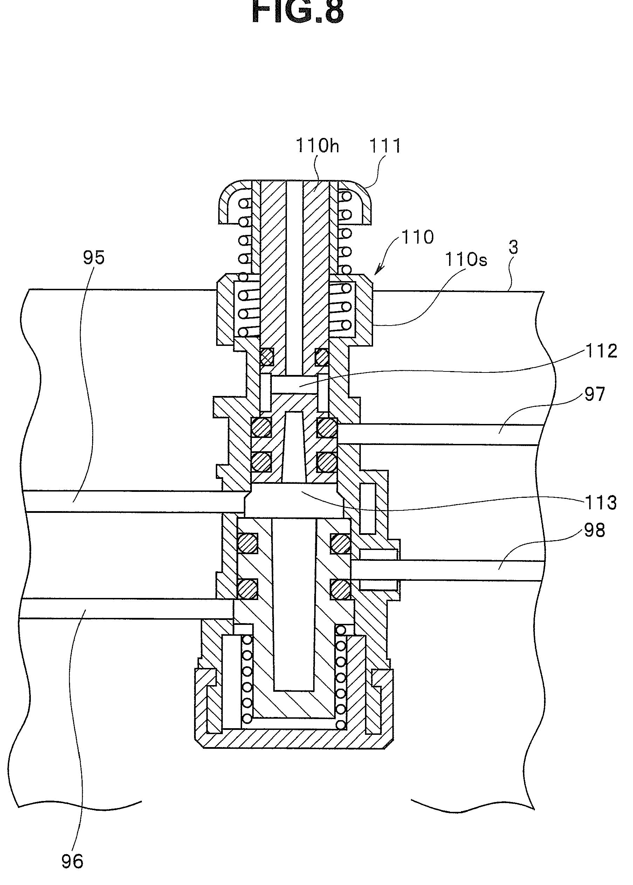

Fig. 7 is a diagram showing a modification of a conduit configuration for supplying the fluid to the first nozzle portion and the second nozzle portion shown in Fig. 6 together with the distal end side of the insertion portion of the endoscope partially as a cross section. Fig. 8 is a partial sectional view showing an overview of a configuration of an air feeding and water feeding switching device provided in the operation portion of the endoscope in Fig. 1.

As shown in Fig. 5 and Fig. 6, in the distal end of the insertion portion 2, a distal end of an air feeding and liquid feeding conduit 90 inserted through the insertion portion 2 is connected to the first nozzle portion 18. Consequently, the air feeding and liquid feeding conduit 90 communicates with the first opening portion 18k. In the distal end of the insertion portion 2, a distal end of an air feeding and liquid feeding conduit 92 branching from the air feeding and liquid feeding conduit 90 is connected to the second nozzle portion 19. Consequently, the air feeding and liquid feeding conduit 92 communicates with the second opening portion 19k. Note that the air feeding and liquid feeding conduit 90 and the air feeding and liquid feeding conduit 92 are formed to be equal in a channel diameter.

As shown in Fig. 7, in the distal end of the insertion portion 2, a distal end of an air feeding and liquid feeding conduit 91 branching from a distal end of the air feeding and liquid feeding conduit 90 inserted through the insertion portion 2 may be connected to the first nozzle portion 18. Further, as shown in Fig. 7, in the distal end of the insertion portion 2, a distal end of the air feeding and liquid feeding conduit 92 branching from the distal end of the air feeding and liquid feeding conduit 90 may be connected to the second nozzle portion 19. Note that the air feeding and liquid feeding conduit 91 and the air feeding and liquid feeding conduit 92 are formed to be equal in a channel diameter.

In the insertion portion 2, a distal end of an air feeding conduit 95 and a distal end of a liquid feeding conduit 96 inserted through the insertion portion 2 and the operation portion 3 are connected to a proximal end of the air feeding and liquid feeding conduit 90. Note that, in the operation portion 3, a proximal end of the air feeding conduit 95 and a proximal end of the liquid feeding conduit 96 are connected to a distal end side region in the inserting direction S of a cylinder 110s of an air feeding and water feeding switching device 110 provided in the operation portion 3.

In the operation portion 3, a distal end of an air feeding conduit 97 and a distal end of a liquid feeding conduit 98 are connected to a proximal end side region in the inserting direction S of the cylinder 110s of the air feeding and water feeding switching device 110. The air feeding conduit 97 and the liquid feeding conduit 98 are inserted through the operation portion 3, the universal cord 4, and the connector 5. Note that a proximal end of the air feeding conduit 97 is connected to a gas supply source in the connector 5. A proximal end of the liquid feeding conduit 98 is connected to a liquid supply source in the connector 5.

As shown in Fig. 8, the water feeding switching device 110 includes, in the cylinder 11 0s a main body portion 110h that can be pressed by a press button 111. The main body portion 11 Oh includes two coupling conduits 112 and 113 on an inside.

When the main body portion 110h is in a position shown in Fig. 8, the water feeding switching device 110 shuts off supply of gas from the air feeding conduit 97 to the air feeding conduit 95 and shuts off supply of liquid from the liquid feeding conduit 98 to the liquid feeding conduit 96.

When the press button 111 is pressed and the main body portion 11 Oh is in a position pushed in downward from the position shown in Fig. 8 by a first distance, the water feeding switching device 110 causes the air feeding conduit 97 and the air feeding conduit 95 to communicate with each other via the coupling conduit 112.

Consequently, the water feeding switching device 110 functions such that the gas is supplied to the air feeding conduit 95 from the air feeding conduit 97 via the coupling conduit 112.

Further, when the press button 111 is further pressed and the main body portion 11 Oh is in a position pushed in downward from the position shown in Fig. 8 by a second distance longer than the first distance, the water feeding switching device 110 causes the air feeding conduit 97 and the air feeding conduit 95 to communicate with each other via the coupling conduit 112. Further, the water feeding switching device 110 causes the liquid feeding conduit 98 and the liquid feeding conduit 96 to communicate with each other via the coupling conduit 113.

Consequently, the water feeding switching device 110 functions such that the gas is supplied to the air feeding conduit 95 from the air feeding conduit 97 via the coupling conduit 112. The water feeding switching device 110 functions such that liquid is supplied to the liquid feeding conduit 96 from the liquid feeding conduit 98 via the coupling conduit 113.

Note that, since the configuration of the water feeding switching device 110 is well-known, more detailed explanation of the configuration is omitted.

Examples of the gas forming the fluid R supplied to the first nozzle portion 18 and the second nozzle portion 19 from the gas supply source via the air feeding conduit 97, the coupling conduit 112, the air feeding conduit 95, and the air feeding and liquid feeding conduit 90 include air.

Further, examples of the liquid forming the fluid R supplied to the first nozzle portion 18 and the second nozzle portion 19 from the liquid supply source via the liquid feeding conduit 98, the coupling conduit 113, the liquid feeding conduit 96, and the air feeding and liquid feeding conduit 90 include liquid having biocompatibility such as sterilized water and saline.

As explained above, in the configuration shown in Fig. 5 and Fig. 6, the distal end of the air feeding and liquid feeding conduit 90 is connected to the first nozzle portion 18. The distal end of the air feeding and liquid feeding conduit 92 branching from the air feeding and liquid feeding conduit 90 is connected to the second nozzle portion 19. Further, respective distal ends of the air feeding conduit 95 and the liquid feeding conduit 96 are connected to a proximal end of the air feeding and liquid feeding conduit 90.

In the configuration shown in Fig. 7, the distal end of the air feeding and liquid feeding conduit 91 branching from the air feeding and liquid feeding conduit 90 is connected to the first nozzle portion 18. The distal end of the air feeding and liquid feeding conduit 92 branching from the air feeding and liquid feeding conduit 90 is connected to the second nozzle portion 19. Further, the respective distal ends of the air feeding conduit 95 and the liquid feeding conduit 96 are connected to the proximal end of the air feeding and liquid feeding conduit 90.

Accordingly, in the present embodiment, an air feeding conduit for supplying the gas to the first nozzle portion 18 and an air feeding conduit for supplying the gas to the second nozzle portion 19 are standardized as the air feeding conduit 95. A liquid feeding conduit for supplying the liquid to the first nozzle portion 18 and a liquid feeding conduit for supplying the liquid to the second nozzle portion 19 are standardized as the liquid feeding conduit 96.

Consequently, the number of conduits inserted through the insertion portion 2 is smaller than when the air feeding conduits and the water feeding conduits are provided separately for the nozzle portions 18 and 19 and the like. Therefore, it is possible to attain a reduction in a diameter of the insertion portion 2. That is, a fluid supply mechanism to the respective nozzle portions 18 and 19 can be configured by an inexpensive and simple configuration.

The air feeding and water feeding switching device 110 is configured be capable of simultaneously performing supply and shut-off of the supply of the gas to the first nozzle portion 18 and the second nozzle portion 19 via the air feeding conduit 95. Further, the air feeding and water feeding switching device 110 is configured be capable of simultaneously performing control of supply and shut-off of the supply of the gas and the liquid to the first nozzle portion 18 and the second nozzle portion 19 via the air feeding conduit 95 and the liquid feeding conduit 96.

That is, when the supply of the gas or the supply of the gas and the liquid is instructed by pressing operation of the press button 111, the gas or the gas and the liquid simultaneously jet from the opening portion 18k of the first nozzle portion 18 and the opening portion 19k of the second nozzle portion 19.

In addition to the above, according to three configurations explained below, the fluid is supplied to the first objective lens 11 and the second objective lens 12 at same speed or a same flow rate per unit time. Note that as a first configuration among the three configurations, as explained above, a≈b or a≈b. As a second configuration, the distal ends of the air feeding conduit 95 and the liquid feeding conduit 96 are connected to the air feeding and liquid feeding conduit 90. Further, as a third configuration, the distal end of the air feeding and liquid feeding conduit 90 or the air feeding and liquid feeding conduit 91 branching from the distal end is connected to the first nozzle portion 18 and the distal end of the air feeding and liquid feeding conduit 92 branching from the air feeding and liquid feeding conduit 90 is connected to the second nozzle portion 19.

Note that, in the present embodiment, as a configuration for simultaneously jetting the gas or the gas and the liquid from the opening portion 18k of the first nozzle portion 18 and the opening portion 19k of the second nozzle portion 19, in order to simplify the configuration, one air feeding and water feeding switching device 110 is used.

The configuration is not limited to the above. An air feeding and water feeding switching device that controls the supply and the shut-off of the supply of the gas or the gas and the liquid may be provided for each air feeding and water feeding conduit connected to the first nozzle portion 18 and each air feeding and water feeding conduit connected to the second nozzle portion 19. In this configuration, the supply and the shut-off of the supply of the gas or the gas and the liquid to the first nozzle portion 18 and the second nozzle portion 19 are simultaneously controlled for each air feeding and water feeding switching device.

However, in the present embodiment, in the configuration in which one air feeding and water feeding switching device 110 is used, a supply mechanism of the fluid to the respective nozzle portions 18 and 19 can be more inexpensively and easily configured.

In this way, in the present embodiment, on the distal end face 2s, the first nozzle portion 18 and the second nozzle portion 19 are provided apart from the first objective lens 11 and the second objective lens 12 in the up direction.

On the distal end face 2s, the first opening portion 18k and the second opening portion 19k are located in parallel to each other.

Further, the direction in which the fluid R is supplied to the lens surface 12m from the second opening portion 19k coincides with the supply direction W of the fluid supplied to the lens surface 11m from the first opening portion 18k.

Consequently, the fluid R is supplied to both of the lens surface 11m of the first objective lens 11 and the lens surface 12m of the second objective lens 12 from the up direction: Therefore, the fluid R flows from the up direction to the down direction.

That is, as shown in Fig. 3, the observer observing the display unit 52 with the dedicated stereoscopic vision glasses on can observe the display unit 52 in a state in which the fluid is supplied from the up direction in both of the first observation image 201 observed by the right eye and the second observation image 202 observed by the left eye.

Accordingly, during the observation of the region to be examined, even while stains and fog of the lens surfaces 11m and 12m are removed by supplying the fluid R to the lens surface 11m of the first objective lens 11 and the lens surface 12m of the second objective lens 12, supply directions of the fluid flowing on the lens surfaces 11m and 12m look the same in the first observation image 201 and the second observation image. Consequently, the region to be examined looks the same in the respective observation images 201 and 202. Accordingly, the observer does not feel a sense of discomfort concerning the respective observation images 201 and 202 and the eyes of the observer do not get tired.

In the present embodiment, further, the distance "a" between the first opening portion 18k and the optical center 11c of the first objective lens 11 is equal to or substantially equal to the distance "b" between the second opening portion 19k and the optical center 12c of the second objective lens 12.

Further, the flow rate of the fluid R per unit time supplied to the lens surface 11m of the first objective lens 11 from the first opening portion 18k and the flow rate of the fluid R per unit time supplied to the lens surface 12m of the second objective lens 12 from the second opening portion 19k are set to be equal or substantially equal.

Consequently, a same amount of the fluid is supplied at same speed to both of the lens surface 11m of the first objective lens 11 and the lens surface 12m of the second objective lens 12 from the up direction.

That is, as shown in Fig. 3, the observer observing the display unit 52 with the dedicated stereoscopic vision glasses on can observe the display unit 52 in a state in which the fluid is supplied in the same amount and at the same speed from the up direction in both of the first observation image 201 observed by the right eye and the second observation image 202 observed by the left eye.

Accordingly, during the observation of the region to be examined, even while stains and fog of the lens surfaces 11m and 12m are removed by supplying the fluid R to the lens surface 11m of the first objective lens 11 and the lens surface 12m of the second objective lens 12, supply directions, supply speeds, and supply amounts of the fluid flowing on the lens surfaces 11m and 12m look the same in the first observation image 201 and the second observation image.

Consequently, the region to be examined looks the same in the respective observation images 201 and 202. Accordingly, the observer does not feel a sense of discomfort concerning the respective observation images 201 and 202 and the eyes of the observer do not get tired.

It is possible to surely supply the fluid R to the first objective lens 11 and the second objective lens 12. Therefore, it is possible to surely remove stains and fog of the lens surfaces 11m and 12m.

Therefore, it is possible to provide the stereoscopic endoscope 1 with which the observer can easily observe the observation images 201 and 202 even when stains and fog of the lens surfaces 11m and 12m of the respective objective lenses 11 and 12 are removed by the supply of the fluid R during the observation of the respective observation images 201 and 202.

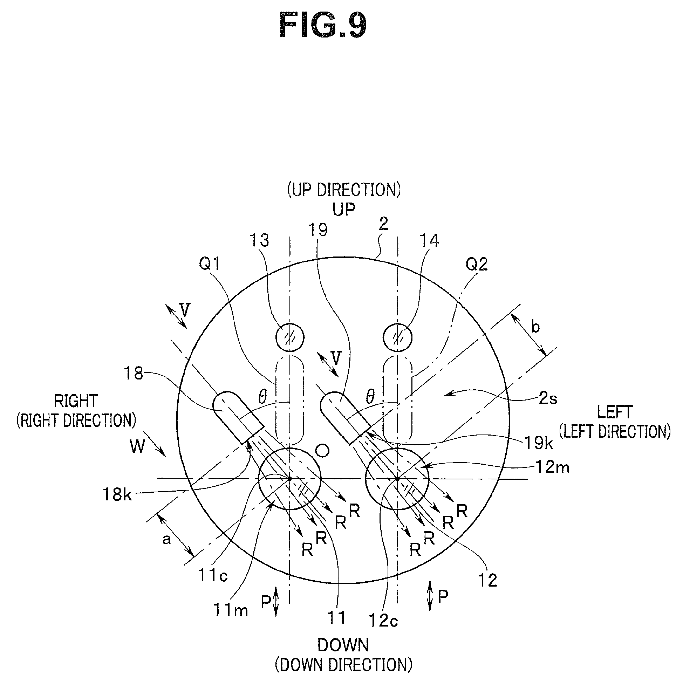

Note that a modification is explained below with reference to Fig. 9. Fig. 9 is an enlarged plan view of the distal end face of the insertion portion showing a modification in which, on the distal end face shown in Fig. 4, the first nozzle portion and the second nozzle portion are located by being shifted in a direction crossing, at a set angle, a direction connecting a side in the up direction and a side in the down direction of the first image pickup device and the second image pickup device.

Note that, in this modification, as shown in Fig. 9, on the distal end face 2s, it is assumed that the first illumination lens 13 and the second illumination lens 14 are respectively provided in positions apart from the first objective lens 11 and the second objective lens 12 further upward than the first nozzle portion 18 and the second nozzle portion 19.

In the present embodiment explained above, on the distal end face 2s, the first opening portion 18k of the first nozzle portion 18 and the second opening portion 19k of the second nozzle portion 19 are respectively provided apart from the first objective lens 11 and the second objective lens 12 in the up direction by "a" and "b" and a=b or a≈b.

However, the first opening portion 18k and the second opening portion 19k are not limited to this. As shown in Fig. 9, when the distal end face 2s is viewed in plan, the first opening portion 18k of the first nozzle portion 18 may be located by being shifted along a direction V crossing the direction P by a set angle θ from a region Q1 between the first objective lens 11 and the first illumination lens 13.

When the distal end face 2s is viewed in plan, the second opening 19k of the second nozzle portion 19 may be located by being shifted along the direction V crossing the direction P by the set angle θ from a region Q2 between the second objective lens 12 and the second illumination lens 14.

Note that in this modification, as in the present embodiment, the opening portion 18k of the first nozzle portion 18 and the opening portion 19k of the second nozzle portion 19 are respectively provided apart from the respective optical centers 11c and 12c of the first objective lens 11 and the second objective lens 12 by "a" and "b" in the direction V and a=b or a≈b.

In this modification, as in the present embodiment, on the distal end face 2s, an opening axis direction of the first opening portion 18k and an opening axis direction of the second opening portion 19k are located in parallel to each other.

Consequently, a direction in which the fluid R is supplied to the lens surface 12m from the second opening portion 19k coincides with the supply direction W of the fluid supplied to the lens surface 11m from the first opening portion 18k.

That is, in the present embodiment, the supply direction W of the fluid R coincides with a direction parallel to the direction V crossing, at the set angle θ, the direction P connecting the side in the up direction and the side in the down direction of the first image pickup device 31 and the second image pickup device 32. Note that the other components are the same as the components explained in the present embodiment.

Effects same as the effects in the present embodiment can be obtained by such a configuration. A degree of freedom of an arrangement layout of the first nozzle portion 18 and the second nozzle portion 19 on the distal end face 2s is improved.

Further, since the first nozzle portion 18 and the second nozzle portion 19 are respectively located by being shifted along the direction V from the regions Q1 and Q2, the respective nozzle portions 18 and 19 can be arranged in an empty region on the distal end face 2s. Accordingly, the insertion portion 2 can be reduced in diameter in the direction P. Therefore, it is possible to realize a reduction in the diameter of the insertion portion 2 more than in the present embodiment.

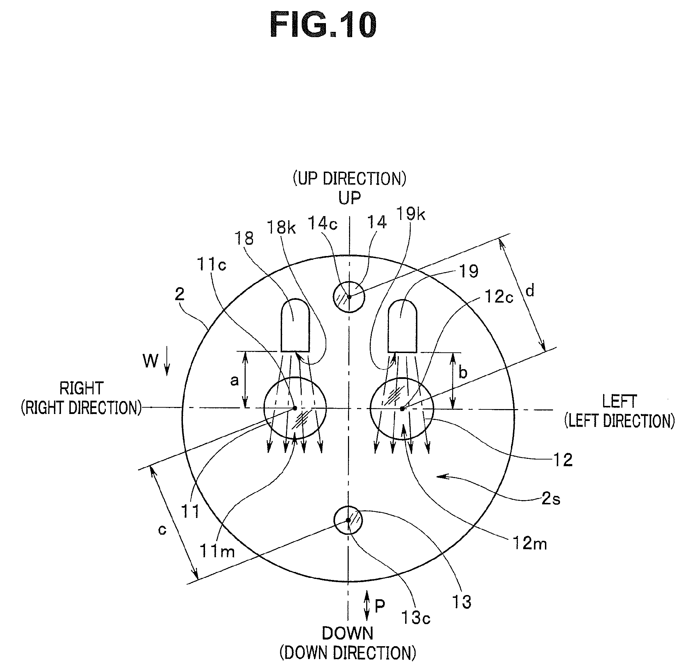

Note that another modification is explained below with reference to Fig. 10. Fig. 10 is an enlarged plan view of the distal end face of the insertion portion showing a modification in which arrangement positions of a first illumination lens and a second illumination lens on the distal end face shown in Fig. 4 are determined with reference to a first objective lens and a second objective lens.

As shown in Fig. 10, in this modification, as in the present embodiment, on the distal end face 2s, the opening portion 18k of the first nozzle portion 18 and the opening portion 19k of the second nozzle portion 19 are respectively provided apart from the respective optical centers 11c and 12c of the first objective lens 11 and the second objective lens 12 by the distances "a" and "b" in the up direction and a=b or a≈b.

In this modification, as in the present embodiment, on the distal end face 2s, the first opening portion 18k and the second opening portion 19k are located in parallel to each other. Consequently, a direction in which the fluid R is supplied to the lens surface 12m from the second opening portion 19k coincides with the supply direction W of the fluid supplied to the lens surface 11m from the first opening portion 18k.

However, in this modification, on the distal end face 2s, the first illumination lens 13 and the second illumination lens 14 are located on a bisector of the respective centers 11 c and 12c along the direction P perpendicular to a line connecting the optical center 11c of the first objective lens 11 and the optical center 12c of the second objective lens 12.

On the distal end face 2s, the first illumination lens 13 is provided below the respective objective lenses 11 and 12. The second illumination lens 14 is provided above the respective objective lenses 11 and 12.

Further, the first illumination lens 13 is provided in a position where a distance between a center 13c of the first illumination lens 13 and the optical center 11c of the first objective lens 11 is a distance "c". The second illumination lens 14 is provided in a position where a distance between a center 14c of the second illumination lens 14 and the optical center 12c of the second objective lens 12 is a distance "d". Note that the distance "c" coincides with the distance "d" (c=d) or substantially coincides with the distance "d" (c≈d). Note that the other components are the same as the components in the present embodiment explained above.

Effects same as the effects of the present embodiment can be obtained by such a configuration. Further, lights irradiated on the inside of the subject from the respective illumination lenses 13 and 14 and reflected by the subject are made incident on the respective objective lenses 11 and 12 at substantially the same light amounts and angles.

Accordingly, since brightness looks the same in the first observation image 201 and the second observation image, the region to be examined looks the same in the respective observation images 201 and 202. Note that the other effects are the same as the effects in the present embodiment.

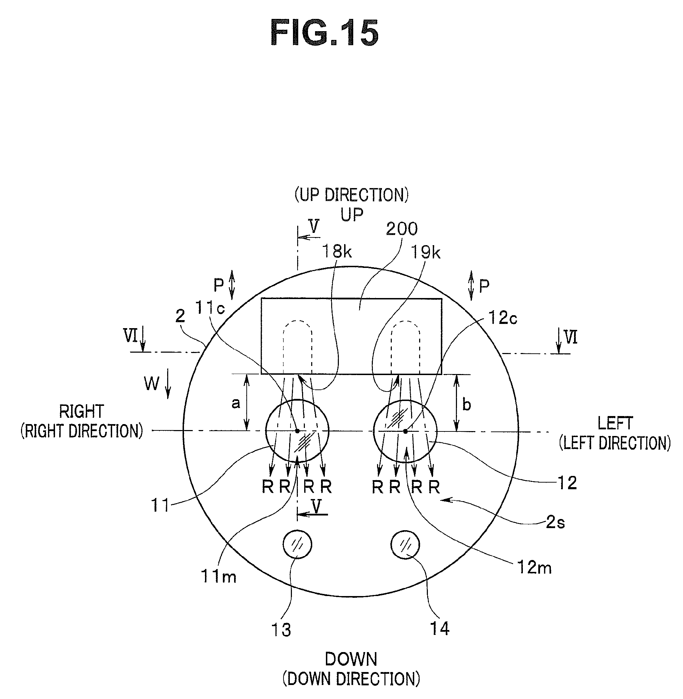

Note that another modification is explained below with reference to Fig. 15. Fig. 15 is an enlarged plan view of the distal end face of the insertion portion showing a modification in which the first opening portion and the second opening portion shown in Fig. 4 are provided in one nozzle portion located on the distal end face of the insertion portion.

In the present embodiment explained above, the first opening portion 18k is provided in the first nozzle portion 18 provided on the distal end face 2s and the second opening portion 19k is provided in the second nozzle portion 19 provided on the distal end face 2s.

The first opening portion 18k and the second opening portion 19k are not limited to this. As shown in Fig. 15, the first opening portion 18k and the second opening portion 19k may be provided in one nozzle portion 200 provided on the distal end face 2s.

Note that, in this case, as in the present embodiment, the distance "a" between the optical axis center 11c and the first opening portion 18k and the distance "b" between the optical axis center 12c and the second opening portion 19k are equal or substantially equal.

The distal ends of the air feeding and liquid feeding conduits 90 and 92 shown in Fig. 5 and Fig. 6 are connected to the nozzle portion 200 such that the air feeding and liquid feeding conduit 90 communicates with the first opening portion 18k and the air feeding and liquid feeding conduit 92 communicates with the opening portion 19k.

Note that, as shown in Fig. 7, the distal end of the air feeding and liquid feeding conduit 91 may be connected to the nozzle 200 to communicate with the first opening portion 18k.

A branching portion of the air feeding and liquid feeding conduits 90 and 92 shown in Figs. 5 and Fig. 6 may be located on an inside of the nozzle 200. Similarly, a branching portion of the air feeding and liquid feeding conduits 91 and 92 shown in Fig. 7 may be located on the inside of the nozzle 200.

With such a configuration, effects same as the effects in the present embodiment explained above can be obtained.

### (Second Embodiment)

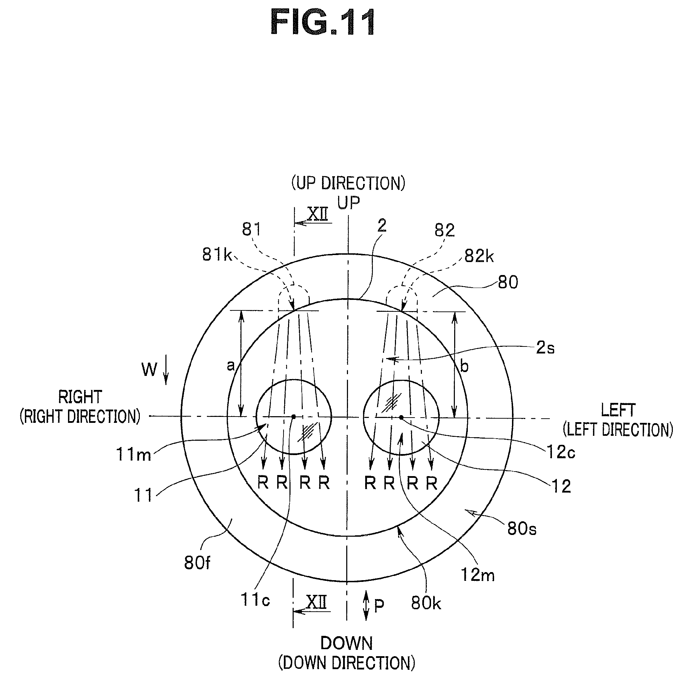

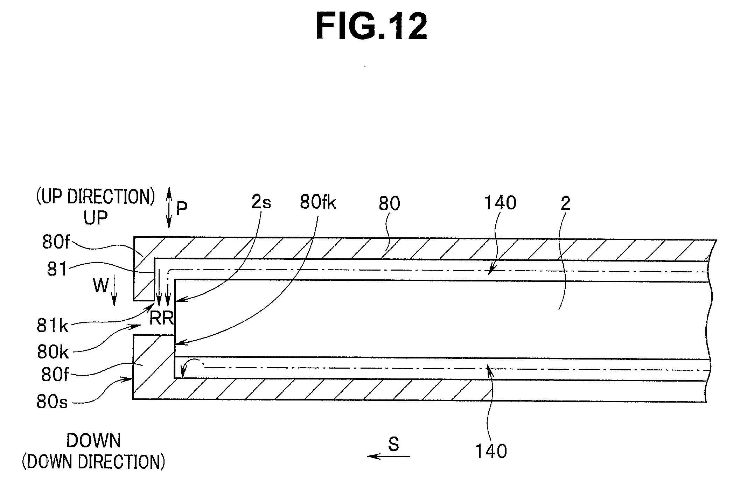

Fig. 11 is a plan view showing a distal end face of an insertion portion of a stereoscopic endoscope of the present embodiment together with a distal end face of a sheath. Fig. 12 is a partial sectional view showing a distal end side of the insertion portion along a XII-XII line in Fig. 11 together with the sheath.

A configuration of the stereoscopic endoscope of the second embodiment is different from the stereoscopic endoscope of the first embodiment shown in Fig. 1 to Fig. 8 in that the first nozzle portion and the second nozzle portion are provided at a distal end of a sheath covering an outer circumference of the insertion portion.

Therefore, only this difference is explained. Components same as the components in the first embodiment are denoted by the same reference numerals and signs and explanation of the components is omitted.

Note that, in the present embodiment, as in the first embodiment, up and down and left and right directions of the first observation image 201 and the second observation image 202 formed on the first image pickup device 31 and the second image pickup device 32 are defined as up and down and left and right directions. In Fig. 12, in order to simplify the drawing, the first objective lens 11 is omitted.

As shown in Fig. 11, a sheath 80 covers an outer circumference of the insertion portion 2 of the stereoscopic endoscope of the present embodiment. Note that a circumferential space 140 is formed between an inner circumferential surface of the sheath 80 and an outer circumferential surface of the insertion portion 2.

A distal end face 80s of the sheath 80 is located in front of the distal end face 2s of the insertion portion 2 in the inserting direction S. At a distal end of the sheath 80, an inward flange 80f extending in an L shape to an inner side in a radial direction of the sheath 80 is circumferentially formed. A proximal end face 80fk at an extension end on a radial direction inner side of the inward flange 80f having the L shape is in contact with a vicinity of an outer circumferential portion on the distal end face 2s.

Note that the respective objective lenses 11 and 12 provided on the distal end face 2s are exposed to a front of the inserting direction S via an opening 80k formed at the distal end of the sheath 80.

In the circumferential inward flange 80f, a first nozzle portion 81 is provided in the up direction of the first objective lens 11. The first nozzle portion 81 supplies the fluid R to the lens surface 11m of the first objective lens 11 from a first opening portion 81k, which is a first delivery portion, located to be opposed to the outer circumference of the first objective lens 11.

Further, in the circumferential inward flange 80f, a second nozzle portion 82 is provided in the up direction of the second objective lens 12. The second nozzle portion 82 supplies the fluid R to the lens surface 12m of the second objective lens 12 from a second opening portion 82k, which is a second delivery portion, located to be opposed to the outer circumference of the second objective lens 12.

Note that a channel communicating with the first opening portion 81k in the first nozzle portion 81 and a channel communicating with the first opening portion 82k in the second nozzle portion 82 communicate with a distal end of a circumferential space 140. That is, the space 140 configures a channel for supplying the fluid R to the first nozzle portion 81 and the second nozzle portion 82.

The space 140 is equivalent to the air feeding and liquid feeding conduit 90 in the first embodiment. That is, the respective distal ends of the air feeding conduit 95 and the liquid feeding conduit 96 are connected to the space 140. In the operation portion 3, as in the first embodiment, the water feeding switching device 110 is provided.

In the present embodiment, the first opening portion 81 k and the second opening portion 82k are located in parallel to each other. The distance "a" of the first opening portion 81k from the optical center 11c of the first objective lens 11 and the distance "b" of the second opening portion 82k from the optical center 12c of the second objective lens 12 coincide with each other (a=b) or substantially coincide with each other (a≈b).

A direction in which the fluid R is supplied to the lens surface 12m from the second opening portion 82k coincides with the supply direction W of the fluid R supplied to the lens surface 11m from the first opening portion 81k.

Consequently, as in the first embodiment, the fluid R is supplied to the lens surface 11m of the first objective lens 11 and the lens surface 12m of the second objective lens 12 from the up direction.

Note that the first nozzle portion 81 and the second nozzle portion 82 may be respectively provided apart from the first objective lens 11 and the second objective lens 12 respectively by the distances "a" and "b" in the down direction in the circumferential inward flange 80f as long as the supply direction W coincides with a direction parallel to the direction P.

Accordingly, in the circumferential inward flange 80f, the first nozzle portion 81 and the second nozzle portion 82 only have to be provided in the up direction or the down direction apart from the first objective lens 11 and the second objective lens 12 respectively by the distances "a" and "b". Note that the other components are the same as the components explained in the first embodiment.

In this way, effects same as the effects in the first embodiment can be obtained even if the first nozzle portion 81 and the second nozzle portion 82 are provided at the distal end of the sheath 80. Since it is unnecessary to provide the respective nozzle portions 18 and 19 on the distal end face 2s, it is possible to attain a reduction in diameter of the insertion portion 2.

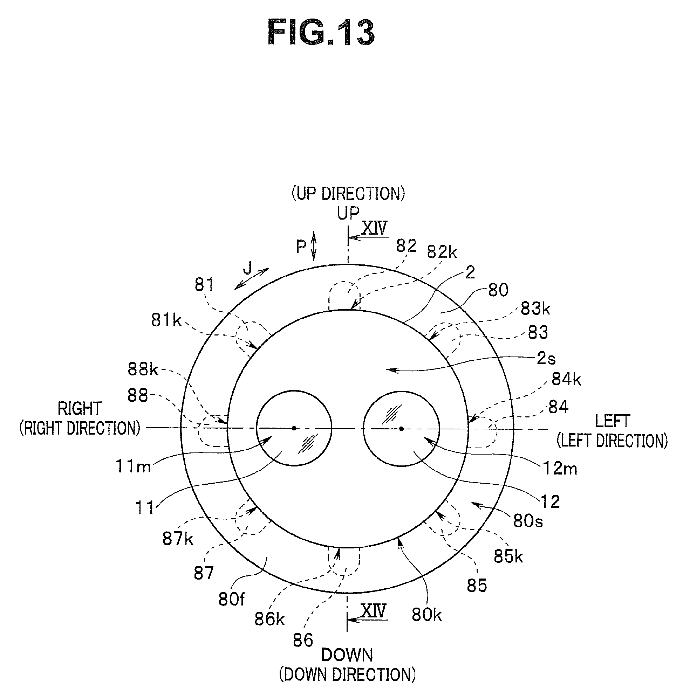

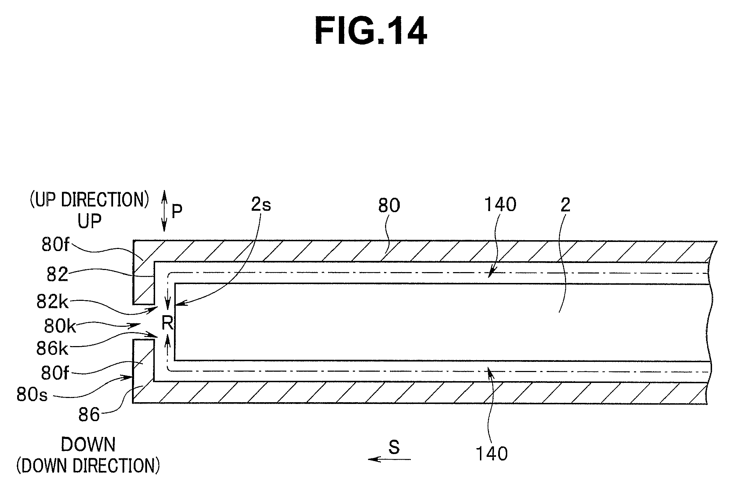

Note that a modification is explained below with reference to Figs. 13 and 14. Fig. 13 is a plan view showing a configuration of a modification of the nozzle portions shown in Fig. 11 together with the distal end face of the insertion portion of the stereoscopic endoscope and the distal end face of the sheath. Fig. 14 is a partial sectional view showing the distal end side of the insertion portion along a XIV-XIV line in Fig. 13 together with the sheath.

In the present embodiment explained above, in the circumferential inward flange 80f, the first nozzle portion 81 that supplies the fluid R to the lens surface 11m of the first objective lens 11 from the first opening portion 81k is provided in the up direction of the first objective lens 11.

Further, in the circumferential inward flange 80f, the second nozzle portion 82 that supplies the fluid R to the lens surface 12m of the second objective lens 12 from the second opening portion 82k is provided in the up direction of the second objective lens 12.

The nozzle portions are not limited to this. A plurality of nozzle portions may be provided at equal intervals in a circumferential direction J in the circumferential inward flange 80f.

Specifically, as shown in Fig. 13 and Fig. 14, in the circumferential direction J, for example, eight nozzle portions may be formed in the circumferential inward flange 80f at equal intervals as a first nozzle portion 81, a second nozzle portion 82, a third nozzle portion 83, a fourth nozzle portion 84, a fifth nozzle portion 85, a sixth nozzle portion 86, a seventh nozzle portion 87, and an eighth nozzle portion 88. Respective opening portions 81k, 82k, 83k, 84, 85k, 86k, 87k, and 88k are opposed to the outer circumferences of the respective objective lenses 11 and 12.

Note that the other components are the same as the components in the present embodiment explained above.

With such a configuration, the fluid R is supplied to the lens surfaces 11m and 12m of the respective objective lenses 11 and 12 from an entire circumferential direction by the respective opening portions 81k to 88k. Therefore, the observer observing the display unit 52 with the dedicated stereoscopic vision glasses on can observe the display unit 52 in a state in which the fluid R is supplied from the entire circumferential direction in both of the first observation image 201 observed by the right eye and the second observation image 202 observed by the left eye.

Accordingly, during the observation of the region to be examined, even while stains and fog of the lens surfaces 11m and 12m are removed by supplying the fluid R to the lens surface 11m of the first objective lens 11 and the lens surface 12m of the second objective lens 12, supply directions of the fluid flowing on the lens surfaces 11m and 12m look substantially the same in the first observation image 201 and the second observation image. Consequently, the region to be examined looks the same in the respective observation images 201 and 202.

Accordingly, the observer does not feel a sense of discomfort concerning the respective observation images 201 and 202 and the eyes of the observer do not get tired. Note that the other effects are the same as the effects in the present embodiment explained above.

Note that, in the first and second embodiments, the stereoscopic endoscope of a front-view type in which the first objective lenses 11 and 12 are provided on the distal end face 2s of the insertion portion 2 is explained as an example. However, the present invention is not limited to this. It goes without saying that the present invention is also applicable to a stereoscopic endoscope of a side-view type in which the objective lenses 11 and 12 are provided on an outer circumferential side surface of the distal end portion of the insertion portion 2.

## Claims

1. A stereoscopic endoscope comprising:
an insertion portion inserted into a subject;
a first observation window (11) provided at a distal end portion on an inserting direction distal end side of the insertion portion;
a second observation window (12) provided at the distal end portion;
a first delivery portion (18, 18k) provided a predetermined distance (a) apart from the optical axis center of the first observation window (11) and configured to deliver fluid (R) to the first observation window; (11) and
a second delivery portion (19,19k) provided a distance (b) equivalent to the predetermined distance (a) apart from the optical axis center of the second observation window (12) and configured to deliver the fluid (R) in a supply direction (W) same as a supply direction of the first delivery portion (18, 18k) to the second observation window (12).

2. The stereoscopic endoscope according to claim 1, wherein
the first delivery portion is provided in a first nozzle portion located at the distal end portion, and
the second delivery portion is provided in a second nozzle portion different from the first nozzle portion located at the distal end portion.

3. The stereoscopic endoscope according to claim 1, wherein the first delivery portion and the second delivery portion are provided in one nozzle portion located at the distal end portion.

4. The stereoscopic endoscope according to claim 1, wherein a flow rate of the fluid per unit time delivered to the first observation window from the first delivery portion is set to be equal to a flow rate of the fluid per unit time delivered to the second observation window from the second delivery portion.

5. The stereoscopic endoscope according to claim 1, wherein
a first air feeding and water feeding conduit for supplying the fluid to the first delivery portion is connected to the first delivery portion and a second air feeding and water feeding conduit for supplying the fluid to the second delivery portion and communicating with the first air feeding and water feeding conduit is connected to the second delivery portion, and
the first air feeding and water feeding conduit and the second air feeding and water feeding conduit are equal in a channel diameter.

6. The stereoscopic endoscope according to claim 1, further comprising:
a first image pickup device located behind the first observation window in the inserting direction in the insertion portion, an image of the region to be examined being formed on the first image pickup device as a first observation image via the first observation window and the first image pickup device defining an up direction of the first observation image;
a second image pickup device located behind the second observation window in the inserting direction in the insertion portion, an image of the region to be examined being formed on the second image pickup device as a second observation image via the second observation window and the second image pickup device defining the up direction of the second observation image, wherein
the first delivery portion is located in, with respect to the first observation window, the up direction defined by the first image pickup device or a down direction of the first observation image defined by the first image pickup device opposite to the up direction, and
the second delivery portion is located in, with respect to the second observation window, the up direction defined by the second image pickup device or a down direction of the second observation image defined by the second image pickup device opposite to the up direction.

7. The stereoscopic endoscope according to claim 6, wherein the supply direction of the fluid delivered to the first observation window from the first delivery portion and the fluid delivered to the second observation window from the second delivery portion is a direction parallel to a direction connecting a side in the up direction and a side the down direction of the first image pickup device and the second image pickup device.

8. The stereoscopic endoscope according to claim 6, wherein the supply direction of the fluid delivered to the first observation window from the first delivery portion and the fluid delivered to the second observation window from the second delivery portion is a direction parallel to a direction crossing, at a set angle, a direction connecting a side in the up direction and a side the down direction of the first image pickup device and the second image pickup device.

9. The stereoscopic endoscope according to claim 8, wherein
at the distal end portion, a first illumination lens that illuminates an inside of the subject is provided in the up direction or the down direction with respect to the first observation window and a second illumination lens that illuminates the inside of the subject is provided in the up direction or the down direction with respect to the second observation window, and
the first delivery portion is located by being shifted along the crossing direction from a region between the first observation window and the first illumination lens and the second delivering unit is located by being shifted along the crossing direction from a region between the second observation window and the second illumination lens when the distal end portion is viewed in plan.

10. The stereoscopic endoscope according to claim 1, wherein the first delivery portion and the second delivery portion are provided at the distal end portion.

11. The stereoscopic endoscope according to claim 1, wherein
a sheath covers an outer circumference of the insertion portion, and
the first delivery portion and the second delivery portion are provided at a distal end of the inserting direction of the sheath.

## Patentansprüche

1. Stereoskopisches Endoskop, das umfasst:
einen Einführabschnitt, der in ein Subjekt eingeführt ist;
ein erstes Beobachtungsfenster (11), das an einem distalen Endabschnitt an einer distalen Einführrichtungs-Endseite des Einführabschnitts vorgesehen ist;
ein zweites Beobachtungsfenster (12), das an dem distalen Endabschnitt vorgesehen ist;
einen ersten Zufuhrabschnitt (18, 18k), der in einem vorbestimmten Abstand (a) von dem Zentrum der optischen Achse des ersten Beobachtungsfensters (11) vorgesehen und dazu eingerichtet ist, dem ersten Beobachtungsfenster (11) ein Fluid (R) zuzuführen, und
einen zweiten Zufuhrabschnitt (19, 19k), der in einem zu dem vorbestimmten Abstand äquivalenten Abstand (b) von dem Zentrum der optischen Achse des zweiten Beobachtungsfensters (12) vorgesehen und dazu eingerichtet ist, dem zweiten Beobachtungsfenster (12) das Fluid (R) in einer Zufuhrrichtung (W) zuzuführen, die die gleiche ist, wie eine Zufuhrrichtung des ersten Zufuhrabschnitts (18, 18k).

2. Stereoskopisches Endoskop nach Anspruch 1, wobei
der erste Zufuhrabschnitt in einem ersten Düsenabschnitt vorgesehen ist, der an dem distalen Endabschnitt angeordnet ist, und
der zweite Zufuhrabschnitt in einem zweiten Düsenabschnitt vorgesehen ist, der sich von dem an dem distalen Endabschnitt angeordneten ersten Düsenabschnitt unterscheidet.

3. Stereoskopisches Endoskop nach Anspruch 1, wobei der erste Zufuhrabschnitt und der zweite Zufuhrabschnitt in einem Düsenabschnitt vorgesehen sind, der an dem distalen Endabschnitt angeordnet ist.

4. Stereoskopisches Endoskop nach Anspruch 1, wobei eine Strömungsrate des dem ersten Beobachtungsfenster von dem ersten Zufuhrabschnitt pro Zeiteinheit zugeführten Fluids so festgelegt ist, dass sie gleich einer Strömungsrate des dem zweiten Beobachtungsfenster von dem zweiten Zufuhrabschnitt pro Zeiteinheit zugeführten Fluids ist.

5. Stereoskopisches Endoskop nach Anspruch 1, wobei
eine erste Luftzufuhr- und Wasserzufuhrleitung zum Zuführen des Fluids zu dem ersten Zufuhrabschnitt mit dem ersten Zufuhrabschnitt verbunden ist und eine zweite Luftzufuhr- und Wasserzufuhrleitung zum Zuführen das Fluid zu dem zweiten Zufuhrabschnitt, die mit der ersten Luftzufuhr- und Wasserzufuhrleitung kommuniziert, mit dem zweiten Zufuhrabschnitt verbunden ist, und
die erste Luftzufuhr- und Wasserzufuhrleitung und die zweite Luftzufuhr- und Wasserzufuhrleitung einen gleichen Kanaldurchmesser aufweisen.

6. Stereoskopisches Endoskop nach Anspruch 1, das ferner umfasst:
eine erste Bildaufnahmeeinrichtung, die hinter dem ersten Beobachtungsfenster in der Einführrichtung des Einführabschnitts angeordnet ist, wobei ein Bild des zu untersuchenden Bereichs als ein erstes Beobachtungsbild über das erste Beobachtungsfenster auf der ersten Bildaufnahmeeinrichtung ausgebildet wird und die erste Bildaufnahmeeinrichtung eine Aufwärtsrichtung des ersten Beobachtungsbilds definiert;
eine zweite Bildaufnahmeeinrichtung, die hinter dem zweiten Beobachtungsfenster in der Einführrichtung des Einführabschnitts angeordnet ist, wobei ein Bild des zu untersuchenden Bereichs als ein zweites Beobachtungsbild über das zweite Beobachtungsfenster auf der zweiten Bildaufnahmeeinrichtung ausgebildet wird und die zweite Bildaufnahmeeinrichtung die Aufwärtsrichtung des zweiten Beobachtungsbilds definiert, wobei
der erste Zufuhrabschnitt bezüglich des ersten Beobachtungsfensters in der durch die erste Bildaufnahmeeinrichtung definierten Aufwärtsrichtung oder einer Abwärtsrichtung des ersten Beobachtungsbilds angeordnet ist, die durch die erste Bildaufnahmeeinrichtung als der Aufwärtsrichtung entgegengerichtet definiert ist, und
der zweite Zufuhrabschnitt bezüglich des zweiten Beobachtungsfensters in der durch die zweite Bildaufnahmeeinrichtung definierten Aufwärtsrichtung oder eine Abwärtsrichtung des zweiten Beobachtungspilz angeordnet ist, die durch die zweite Bildaufnahmeeinrichtung als der Aufwärtsrichtung entgegengerichtet definiert ist.

7. Stereoskopisches Endoskop nach Anspruch 6, wobei die Zufuhrrichtung des dem ersten Beobachtungsfenster von dem ersten Zufuhrabschnitt zugeführten Fluids und des dem zweiten Beobachtungsfenster von dem zweiten Zufuhrabschnitt zugeführten Fluids eine Richtung ist, die parallel zu einer Richtung ist, die eine Seite in der Aufwärtsrichtung und eine Seite in der Abwärtsrichtung der ersten Bildaufnahmeeinrichtung und der zweiten Bildaufnahmeeinrichtung verbindet.

8. Stereoskopisches Endoskop nach Anspruch 6, wobei die Zufuhrrichtung des dem ersten Beobachtungsfenster von dem ersten Zufuhrabschnitt zugeführten Fluids und des dem zweiten Beobachtungsfenster von dem zweiten Zufuhrabschnitt zugeführten Fluids eine Richtung ist, die parallel zu einer Richtung ist, die in einem festgelegten Winkel eine Richtung schneidet, die eine Seite in der Aufwärtsrichtung und eine Seite in der Abwärtsrichtung der ersten Bildaufnahmeeinrichtung und der zweiten Bildaufnahmeeinrichtung verbindet.

9. Stereoskopisches Endoskop nach Anspruch 8, wobei
an dem distalen Endabschnitt eine erste Beleuchtungslinse, die ein Inneres des Subjekts beleuchtet, in der Aufwärtsrichtung oder der Abwärtsrichtung bezüglich des ersten Beobachtungsfensters vorgesehen ist und eine zweite Beobachtungslinse, die das Innere des Subjekts beleuchtet, in der Aufwärtsrichtung oder der Abwärtsrichtung bezüglich des zweiten Beobachtungsfensters vorgesehen ist, und
der erste Zufuhrabschnitt angeordnet ist, indem er entlang der Kreuzungsrichtung von einem Bereich zwischen dem ersten Beobachtungsfenster und der ersten Beleuchtungslinse verschoben ist, und die zweite Zufuhreinheit angeordnet ist, indem sie entlang der Kreuzungsrichtung von einem Bereich zwischen dem zweiten Beobachtungsfenster und der zweiten Beleuchtungslinse verschoben ist, wenn der distale Endabschnitt in einer Draufsicht betrachtet wird.

10. Stereoskopisches Endoskop nach Anspruch 1, wobei der erste Zufuhrabschnitt und der zweite Zufuhrabschnitt an dem distalen Endabschnitt vorgesehen sind.

11. Stereoskopisches Endoskop nach Anspruch 1, wobei
eine Hülse einen Außenumfang des Einführabschnitts abdeckt und
der erste Zufuhrabschnitt und der zweite Zufuhrabschnitt an einem distalen Ende der Einführrichtung der Hülse vorgesehen sind.

## Revendications

1. Endoscope stéréoscopique comprenant :
une partie d'insertion insérée dans un sujet ;
une première fenêtre d'observation (11) prévue au niveau d'une partie d'extrémité distale sur un côté d'extrémité distale dans le sens d'insertion de la partie d'insertion ;
une deuxième fenêtre d'observation (12) prévue au niveau de la partie d'extrémité distale ;
une première partie d'alimentation (18, 18k) prévue espacée d'une distance prédéterminée (a) du centre de l'axe optique de la première fenêtre d'observation (11) et configurée pour délivrer un fluide (R) vers la première fenêtre d'observation (11); et
une deuxième partie d'alimentation (19, 19k) prévue espacée d'une distance (b) équivalente à la distance prédéterminée (a) du centre de l'axe optique de la deuxième fenêtre d'observation (12) et configurée pour délivrer le fluide (R) dans une direction d'alimentation (W) identique à une direction d'alimentation de la première partie d'alimentation (18, 18k) vers la deuxième fenêtre d'observation (12).

2. Endoscope stéréoscopique selon la revendication 1, dans lequel
la première partie d'alimentation est prévue dans une première partie de buse placée au niveau de la partie d'extrémité distale, et
la deuxième partie d'alimentation est prévue dans une deuxième partie de buse différente de la première partie de buse placée au niveau de la partie d'extrémité distale.

3. Endoscope stéréoscopique selon la revendication 1, dans lequel la première partie d'alimentation et la deuxième partie d'alimentation sont prévues dans une partie de buse placée au niveau de la partie d'extrémité distale.

4. Endoscope stéréoscopique selon la revendication 1, dans lequel un débit du fluide par temps unitaire délivré vers la première fenêtre d'observation depuis la première partie d'alimentation est établi pour être égal à un débit du fluide par temps unitaire délivré vers la deuxième fenêtre d'observation depuis la deuxième partie d'alimentation.

5. Endoscope stéréoscopique selon la revendication 1, dans lequel
un premier conduit d'alimentation en air et d'alimentation en eau destiné à délivrer le fluide vers la première partie d'alimentation est connecté à la première partie d'alimentation et un deuxième conduit d'alimentation en air et d'alimentation en eau destiné à délivrer le fluide vers la deuxième partie d'alimentation et communiquant avec le premier conduit d'alimentation en air et d'alimentation en eau est connecté à la deuxième partie d'alimentation, et
le premier conduit d'alimentation en air et d'alimentation en eau et le deuxième conduit d'alimentation en air et d'alimentation en eau présentent un diamètre de canal égal.

6. Endoscope stéréoscopique selon la revendication 1, comprenant en outre :
un premier dispositif de capture d'image placé derrière la première fenêtre d'observation dans la direction d'insertion dans la partie d'insertion, une image de la région devant être examinée étant formée sur le premier dispositif de capture d'image en tant que première image d'observation via la première fenêtre d'observation et le premier dispositif de capture d'image définissant une direction vers le haut de la première image d'observation ;
un deuxième dispositif de capture d'image placé derrière la deuxième fenêtre d'observation dans la direction d'insertion dans la partie d'insertion, une image de la région devant être examinée étant formée sur le deuxième dispositif de capture d'image en tant que deuxième image d'observation via la deuxième fenêtre d'observation et le deuxième dispositif de capture d'image définissant la direction vers le haut de la deuxième image d'observation, dans lequel
la première partie d'alimentation est placée, par rapport à la première fenêtre d'observation, dans la direction vers le haut définie par le premier dispositif de capture d'image ou dans une direction vers le bas de la première image d'observation définie par le premier dispositif de capture d'image à l'opposé de la direction vers le haut, et
la deuxième partie d'alimentation est placée, par rapport à la deuxième fenêtre d'observation, dans la direction vers le haut définie par le deuxième dispositif de capture d'image ou dans une direction vers le bas de la deuxième image d'observation définie par le deuxième dispositif de capture d'image à l'opposé de la direction vers le haut.

7. Endoscope stéréoscopique selon la revendication 6, dans lequel la direction d'alimentation du fluide délivré vers la première fenêtre d'observation depuis la première partie d'alimentation et du fluide délivré vers la deuxième fenêtre d'observation depuis la deuxième partie d'alimentation est une direction parallèle à une direction connectant un côté dans la direction vers le haut et un côté dans la direction vers le bas du premier dispositif de capture d'image et du deuxième dispositif de capture d'image.

8. Endoscope stéréoscopique selon la revendication 6, dans lequel la direction d'alimentation du fluide délivré vers la première fenêtre d'observation depuis la première partie d'alimentation et du fluide délivré vers la deuxième fenêtre d'observation depuis la deuxième partie d'alimentation est une direction parallèle à une direction croisant, à un angle établi, une direction connectant un côté dans la direction vers le haut et un côté dans la direction vers le bas du premier dispositif de capture d'image et du deuxième dispositif de capture d'image.

9. Endoscope stéréoscopique selon la revendication 8, dans lequel
au niveau de la partie d'extrémité distale, une première lentille d'éclairage qui éclaire l'intérieur du sujet est prévue dans la direction vers le haut ou dans la direction vers le bas par rapport à la première fenêtre d'observation et une deuxième lentille d'éclairage qui éclaire l'intérieur du sujet est prévue dans la direction vers le haut ou dans la direction vers le bas par rapport à la deuxième fenêtre d'observation, et
la première partie d'alimentation est placée en étant décalée le long de la direction de croisement par rapport à une région entre la première fenêtre d'observation et la première lentille d'éclairage et la deuxième unité d'alimentation est placée en étant décalée le long de la direction de croisement par rapport à une région entre la deuxième fenêtre d'observation et la deuxième lentille d'éclairage lorsque la partie d'extrémité distale est vue en plan.

10. Endoscope stéréoscopique selon la revendication 1, dans lequel la première partie d'alimentation et la deuxième partie d'alimentation sont prévues au niveau de la partie d'extrémité distale.

11. Endoscope stéréoscopique selon la revendication 1, dans lequel
une gaine couvre une circonférence externe de la partie d'insertion, et
la première partie d'alimentation et la deuxième partie d'alimentation sont prévues au niveau d'une extrémité distale de la direction d'insertion de la gaine.
